# EUROPEAN PATENT SPECIFICATION

(11) **EP 3 829 699 B1**
(45) Date of publication and mention of the grant of the patent: **25.03.2026**
(21) Application number: 19749158.2
(22) Date of filing: 23.07.2019
(51) Int. Cl.: A61N 1/372, G16H 50/70, A61N 1/08, A61N 1/37

(54) **SYSTEMS FOR PRESENTING PHYSIOLOGIC DATA**
SYSTEME ZUR DARSTELLUNG VON PHYSIOLOGISCHEN DATEN
SYSTÈMES DE PRÉSENTATION DE DONNÉES PHYSIOLOGIQUES

(30) Priority: 03.08.2018 US 201862714407 P
(43) Date of publication of application: 09.06.2021
(73) Proprietor: Cardiac Pacemakers, Inc., St. Paul, Minnesota 55112-5798 (US)
(72) Inventor: PERSCHBACHER, David L., Coon Rapids, Minnesota 55448 (US); SAHA, Sunipa, Shoreview, Minnesota 55126 (US); MAHAJAN, Deepa, North Oaks, Minnesota 55127 (US); BOHN, Derek D., Woodbury, Minnesota 55125 (US)
(74) Representative: Pfenning, Meinig & Partner mbB
(86) International application number: PCT/US2019/042986
(87) International publication number: WO 2020/028094

(56) References cited:
- WO-A1-01/14006
- WO-A1-2009/085074
- WO-A1-2013/048980
- US-A1- 2006 253 042
- US-A1- 2014 236 029

## Description

### TECHNICAL FIELD

This document relates generally to medical devices, and more particularly, to systems, devices and methods for presenting physiologic data to a user.

### BACKGROUND

Implantable medical devices (IMDs) have been used for monitoring patient health condition or disease states and delivering therapies. For example, implantable cardioverter-defibrillators (ICDs) are used to monitor certain abnormal heart rhythms. Some IMDs may be used to monitor progression of a chronic disease, such as worsening of cardiac performance due to congestive heart failure (CHF). In addition to diagnostic capabilities, the IMDs may also provide therapies to treat or alleviate certain medical conditions, such as cardiac electrostimulation therapies to treat cardiac arrhythmia or to rectify cardiac dyssynchrony in CHF patients.

The IMDs may record medical data associated with detected physiologic events such as a cardiac arrhythmia or worsening heart failure (WHF). Some IMDs may register a patient-triggered episode of a physiologic event, and record physiologic data in response to the patient trigger. The IMDs may be interconnected to a patient management system via a data communication network. Device data, such as the medical data associated with the detected physiologic events, may be transmitted to a patient management system, through which a healthcare professional may remotely follow up with the patients or assess functions of the IMDs on a regular basis. For example, the healthcare provider may review the recorded medical data associated with physiologic event episodes, determine the presence of or possible causes leading to the physiologic event, or assess whether a prescribed therapy has resulted in desired therapeutic outcome.

Document WO 2013/048980 A1 discloses generating and displaying an electrogram (EGM) summary for use by physicians or other clinicians. An implantable medical device (IMD) transmits EGM signal data for a number of cardiac episodes to an external computing device. The external computing device selects a subset of the cardiac episodes for which information or images are displayed to the user. In various examples, cardiac episodes may be selected for display based at least in part on a retrospective analysis classification of the cardiac episode.

Document WO 2009/085074 A1 discloses a cardiac rhythm management (CRM) system that includes an implantable cardioverter defibrillator (ICD) and an external system. The ICD includes a plurality of functional modules performing tachyarrhythmia classification and therapy control functions using atrial tachyarrhythmia rate thresholds that are set to a unified value. In one embodiment, the CRM system allows a user to activate and deactivate each of the functional modules and program the unified value using the external system.

Document US 2006/253042A1 describes methods and systems that are directed to acquiring and organizing information associated with at least one syncope event. A syncope event may be a suspected syncope event, a verified syncope event or a syncope event that is suspected and verified. Automated processes are used to collect information associated with at least one syncope event and organize the information as a syncope log entry. At least one of acquiring the information and organizing the information is performed at least in part implantably.

### OVERVIEW

A patient management system may concurrently manage physiologic event episodes from multiple AMDs. Human review (e.g., by a clinician) and annotation of a large amount of device-recorded physiologic event data, such as cardiac arrhythmia episodes, may require substantial clinical, technical, and human resources, which can be costly or otherwise time consuming for a healthcare facility. For examples, some device-recorded physiologic event episode may contain substantial amount of data, including multiple data channels such as recorded from various electrodes or sensors. Data recorded in some data channels may be as long as 10-30 minutes. It is generally desirable to reduce the amount of data to be presented to a clinician, yet without missing or distorting important information that may be necessary for the clinician to form his/her clinical judgement with regard to patient condition, diagnosis, or therapy outcome efficacy, or an assessment of the system or device functionality.

The physiologic event episodes are generally recorded in response to some triggering events, such as a physiologic trigger (e.g., when a physiologic parameter satisfies a predetermined condition), a patient trigger (e.g., patient voluntarily activates the data recording), or a time trigger (e.g., data recorded periodically or a specific time of a day). Depending on the nature of the triggering event, some device-recorded episodes may not contain an "event of interest." For example, patient-triggered or physiologic sensor-triggered episodes may be false positive (FP) events, meaning that no target events (e.g., cardiac arrhythmia of a particular type) have ever occurred during the time of recording. On the other hand, for some device-recorded episodes that do contain events of interest, data portions that correspond to the events of interest may not be easily identified. For example, for a cardiac arrhythmic event, data portion of interest may include an onset, termination, or a segment of significant diagnostic value (e.g., change in heart rate, or characteristic signal morphology). Conventionally, to identify the event of interest and data portions of interest from the device-recorded episode data, a user would scroll through a large volume of device-recorded episodes. This can be time consuming, and require substantial effort and resources. For at least the reasons set forth above, the present inventors have recognized an unmet need for a system and method to more efficiently present physiologic data of clinical significance, so as to save time, effort, resource, and the cost associated with data review.

### SUMMARY OF THE INVENTION

The invention is defined in claim 1. Advantageous embodiments of the invention are defined in the dependent claims. Any therapeutic methods discussed hereinafter are presented for illustrative purposes only and are not claimed as such.

### SUMMARY OF THE DISCLOSURE

The systems, devices, and methods discussed in this document may improve the performance of medical system for managing device-detected event episodes, such as episodes of arrhythmia, worsening heart failure event, among others. As previously discussed, a challenge in patient management is human time and effort required to review a large amount of device-recorded episode data. The present document provides a solution of automatic recognition, and prioritized presentation, of data portions of clinical significance from the recorded episode according to event-specific signal metrics. Compared to conventional data presentation technology, the automatic recognition, presentation, and prioritization of data subsets as discussed herein may lighten a clinician's time and burden for episode evaluation and adjudication with improved the efficiency. With improved data presentation, a clinician may more quickly effectively recognize FP detections at the expense of less time and effort, such that future inappropriate therapies or medical interventions to the patient may be avoided or reduced. Accordingly, the subject matter discussed herein may better align medical resources to serve those patients with critical medical conditions.

The automatic recognition and presentation of data portions of clinical significance discussed herein may also improve the functionality of a patient management system. The recognition and prioritization of data portions of interest may be performed in a communicator, a mobile monitor, a programmer, or a remote patient management system in communication with an AMD. As such, in some cases, improved alert management may be achieved without modifying existing patient AMDs or physiologic event detectors. In some instances, the recognized data portions of clinical significance may also be prioritized for storage in a memory. More efficient memory usage may be realized than a traditional medical system. With fewer alerts and events for adjudication, complexity and operating cost of the patient management system can be reduced. Additionally, with reduced FP detections, fewer unnecessary device therapy, drugs, and procedures may be scheduled, prescribed, or provided, battery life and longevity of the AMD can be extended, and an overall system cost savings may be realized. Other aspects of the disclosure will be apparent to persons skilled in the art upon reading and understanding the following detailed description and viewing the drawings that form a part thereof, each of which are not to be taken in a limiting sense.

### BRIEF DESCRIPTION OF THE DRAWINGS

Various embodiments are illustrated by way of example in the figures of the accompanying drawings. Such embodiments are demonstrative and not intended to be exhaustive or exclusive embodiments of the present subject matter.
FIG. 1 illustrates an example of a Cardiac Rhythm Management (CRM) system and portions of an environment in which the CRM system may operate.
FIG. 2 illustrates generally an example of a data presentation system for selectively presenting physiologic data to a user.
FIG. 3 illustrates generally an example of a data prioritizer circuit that can prioritize the data subsets taken from a physiologic signal corresponding to device-detected atrial fibrillation episode.
FIG. 4 is a graph illustrating an example of selecting a portion of a heart rate time series for presenting to a user to adjudicate a device-detection of an AF episode.
FIG. 5 illustrates generally an example of a method for presenting physiologic data to a user on a user interface.
FIG. 6 illustrates generally an example of a method for prioritizing the data subsets taken from a physiologic signal corresponding to a device-detected AF episode.
FIG. 7 illustrates generally a block diagram of an example machine upon which any one or more of the techniques (e.g., methodologies) discussed herein may perform.

### DETAILED DESCRIPTION

Disclosed herein are systems, devices, and methods for presenting physiologic data to a user. An exemplary system includes a presentation control circuit that may generate signal metrics for various data subsets of a physiologic signal corresponding to a device-detected presence of a physiologic event, and determine a target subset from the plurality of data subsets. The target set has a corresponding signal metric satisfying a specific condition. The presentation control circuit may prioritize a presentation of the recognized target subset over other data subsets to the user. A user may adjudicate the device detection of the physiologic event, or adjust one or more device parameters.

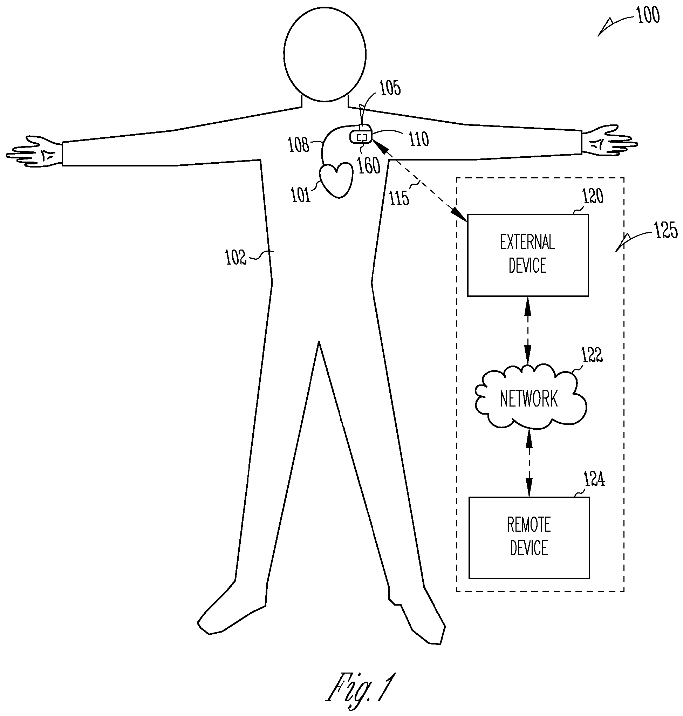

FIG. 1 illustrates generally an example of a Cardiac Rhythm Management (CRM) system 100 and portions of an environment in which the system 100 may operate. The CRM system 100 may include an ambulatory system 105 associated with a patient 102, an external system 125, and a telemetry link 115 providing for communication between the ambulatory system 105 and the external system 125.

The ambulatory system 105 may include an ambulatory medical device (AMD) 110. In an example, the AMD 110 may be an implantable device subcutaneously implanted in a chest, abdomen, or other parts of the patient 102. Examples of the implantable device may include, but are not limited to, pacemakers, pacemaker/defibrillators, cardiac resynchronization therapy (CRT) devices, cardiac remodeling control therapy (RCT) devices, neuromodulators, drug delivery devices, biological therapy devices, diagnostic devices such as cardiac monitors or loop recorders, or patient monitors, among others. The AMD 110 may alternatively or additionally include subcutaneously implanted devices, wearable devices, or other external monitoring or therapeutic medical devices or equipment.

The AMD 110 may be coupled to a lead system 108. The lead system 108 may include one or more transvenously, subcutaneously, or non-invasively placed leads or catheters. Each lead or catheter may include one or more electrodes. The arrangements and uses of the lead system 108 and the associated electrodes may be determined based on the patient need and the capability of the AMD 110. The associated electrodes on the lead system 108 may be positioned at the patient's thorax or abdomen to sense a physiologic signal indicative of cardiac activity, or physiologic responses to diagnostic or therapeutic stimulations to a target tissue. By way of example and not limitation, and as illustrated in FIG. 1, the lead system 108 may be configured to be surgically inserted into, or positioned on the surface of, a heart 101. The electrodes on the lead system 108 may be positioned on a portion of a heart 101, such as a right atrium (RA), a right ventricle (RV), a left atrium (LA), or a left ventricle (LV), or any tissue between or near the heart portions. In some examples, the lead system 108 and the associated electrodes may alternatively be positioned on other parts of the body to sense a physiologic signal containing information about patient ventricular heart rate or pulse rate. In an example, the ambulatory system 105 may include one or more leadless sensors not being tethered to the AMD 110 via the lead system 108. The leadless ambulatory sensors may be configured to sense a physiologic signal and wirelessly communicate with the AMD 110.

The AMD 110 may be configured as a monitoring and diagnostic device. The AMD 110 may include a hermetically sealed can that houses one or more of a sensing circuit, a control circuit, a communication circuit, and a battery, among other components. The sensing circuit may sense a physiologic signal, such as by using a physiologic sensor or the electrodes associated with the lead system 108. Examples of the physiologic signal may include one or more of electrocardiogram, intracardiac electrogram, arrhythmia, heart rate, heart rate variability, intrathoracic impedance, intracardiac impedance, arterial pressure, pulmonary artery pressure, left atrial pressure, RV pressure, LV coronary pressure, coronary blood temperature, blood oxygen saturation, one or more heart sounds, intracardiac acceleration, physical activity or exertion level, physiologic response to activity, posture, respiration rate, tidal volume, respiratory sounds, body weight, or body temperature.

In an example, the AMD 110 may include a cardiac event detector circuit 160 configured to detect a physiologic event, such as cardiac arrhythmia episode, a worsening heart failure (WHF) event from the patient 102. Examples of cardiac arrhythmia include atrial tachyarrhythmia, such as an AFL or AF episode, or ventricular tachyarrhythmia, such as a ventricular tachycardia or ventricular fibrillation episode. The AMD 110 may sense a physiologic signal, detect a cardiac event, and store physiologic data in a memory. The AMD 110 may output the detected cardiac event to a user such as the patient or a clinician, or to a process including, for example, an instance of a computer program executable in a microprocessor. In an example, the process may include an automated generation of recommendations for delivering a therapy, such as anti-arrhythmic therapy, or a recommendation for further diagnostic test or treatment.

The AMD 110 may alternatively be configured as a therapeutic device configured to treat arrhythmia or other heart conditions. The AMD 110 may additionally include a therapy unit that may generate and deliver one or more therapies. The therapy may be delivered to the patient 102 via the lead system 108 and the associated electrodes. The therapies may include electrical, magnetic, or other types of therapy. The therapy may include anti-arrhythmic therapy to treat an arrhythmia or to treat or control one or more complications from arrhythmias, such as syncope, congestive heart failure, or stroke, among others. Examples of the anti-arrhythmic therapy may include pacing, cardioversion, defibrillation, neuromodulation, drug therapies, or biological therapies, among other types of therapies. In an example, the therapies may include cardiac resynchronization therapy (CRT) for rectifying dyssynchrony and improving cardiac function in CHF patients. In some examples, the AMD 110 may include a drug delivery system such as a drug infusion pump to deliver drugs to the patient for managing arrhythmias or complications from arrhythmias.

Although the discussion herein with respect to the AMD 110 focuses on implantable system, this is meant only by way of example and not limitation. It is within the contemplation of the inventors and within the scope of this document, that the systems, devices, and methods discussed herein may also be implemented in, and executed by, a subcutaneous medical device such as a subcutaneous monitor or diagnostic device, wearable devices (e.g., watch-like devices, patch-based devices, or other accessories), or other ambulatory medical devices.

The external system 125 may be communicated with the AMD 110 via a communication link 115. The external system 125 may include a dedicated hardware/software system such as a programmer, a remote server-based patient management system, or alternatively a system defined predominantly by software running on a standard personal computer. The external system 125 may be used to control the operation of the AMD 110. The external system 125 may additionally receive via the communication link 115 information acquired by AMD 110, such as one or more physiologic signals.

By way of example and not limitation, the external system 125 may include an external device 120 in proximity of the AMD 110, a remote device 124 in a location relatively distant from the AMD 110, and a telecommunication network 122 linking the external device 120 and the remote device 124. The telemetry link 115 may be an inductive telemetry link, a capacitive telemetry link, or a radio-frequency (RF) telemetry link. The telemetry link 115 may provide for data transmission from the AMD 110 to the external system 125. This may include, for example, transmitting real-time physiologic data acquired by the AMD 110, extracting physiologic data acquired by and stored in the AMD 110, extracting patient history data such as data indicative of occurrences of arrhythmias, occurrences of decompensation, and therapy deliveries recorded in the AMD 110, and extracting data indicating an operational status of the AMD 110 (e.g., battery status and lead impedance). The telemetry link 115 may also provide for data transmission from the external system 125 to the AMD 110. This may include, for example, programming the AMD 110 to perform one or more of acquiring physiologic data, performing at least one self-diagnostic test (such as for a device operational status), analyzing the physiologic data to detect cardiac arrhythmias, or optionally delivering or adjusting a therapy to the patient 102.

One or more of the external device 120 or the remote device 124 may include a display for displaying the physiologic or functional signals, or alerts, alarms, emergency calls, or other forms of warnings to signal the detection of arrhythmias. In some examples, the external system 125 may include an external data processor configured to analyze the physiologic or functional signals received by the AMD 110, and to confirm or reject the detection of arrhythmias. Computationally intensive algorithms, such as machine-learning algorithms, may be implemented in the external data processor to process the data retrospectively to detect cardia arrhythmias.

In an example, the external data processor may process a physiologic signal received from the AMD 110 by evaluating a signal metric over a plurality of data subsets, such as different signal portions taken from the received physiologic signal. The external data processor may select a signal portion (i.e., a target subset) more representative of a characteristic of the detected cardiac event than other subsets or signal portions (e.g., a portion of ECG or EGM showing high variable ventricular heart rates, a characteristic of an AF episode). The external data processor may prioritize a presentation of the selected signal portion over other subsets of the physiologic data to the user, such as displaying the selected signal portion before displaying other portions of the physiologic signal. By reviewing the selected signal portion of the physiologic signal, the user may use the external device 120 or the remote device 124 to adjudicate a device-detected presence of a physiologic event (e.g., a cardiac arrhythmia), or to adjust one or more parameter used by the AMD 110 to detect the physiologic event, or to adjust therapy delivered to the patient.

Portions of the AMD 110 or the external system 125 may be implemented using hardware, software, firmware, or combinations thereof. Portions of the AMD 110 or the external system 125 may be implemented using an application-specific circuit that may be constructed or configured to perform one or more functions, or may be implemented using a general-purpose circuit that may be programmed or otherwise configured to perform one or more particular functions. Such a general-purpose circuit may include a microprocessor or a portion thereof, a microcontroller or a portion thereof, or a programmable logic circuit, or a portion thereof. For example, a "comparator" may include, among other things, an electronic circuit comparator that may be constructed to perform the specific function of a comparison between two signals or the comparator may be implemented as a portion of a general-purpose circuit that may be driven by a code instructing a portion of the general-purpose circuit to perform a comparison between the two signals.

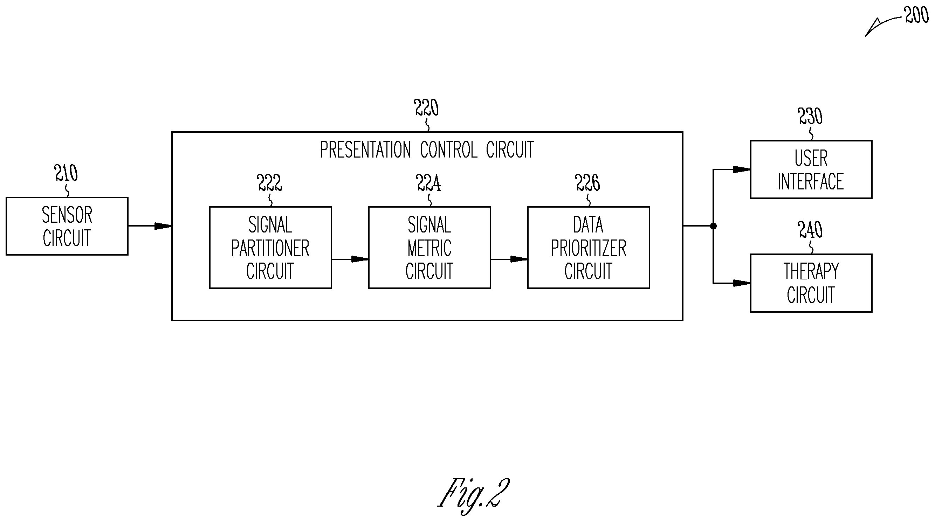

FIG. 2 illustrates generally an example of a data presentation system 200 configured to selectively present physiologic data to a user. Portions of data presentation system 200 may be included in the external system 125, such as the external device 120 or the remote device 124, or distributed between the external system 125 and the AMD 110. The data presentation system 200 may include one or more of a data receiver 210, a presentation control circuit 220, and a user interface unit 230. The data presentation system 200 may be configured as a cardiac monitor or diagnostic device for monitoring patient health status. In some examples, the data presentation system 200 may additionally include an optional therapy circuit 240.

The data receiver 210 may receive physiologic data that is acquired during a physiologic event, such as physiologic signals recorded by the AMD 110. The AMD may sense a physiologic signal using an implantable, wearable, or otherwise ambulatory sensors or electrodes associated with the patient. Examples of the physiologic signals may include surface electrocardiography (ECG) such as sensed from electrodes on the body surface, subcutaneous ECG such as sensed from electrodes placed under the skin, intracardiac electrogram (EGM) sensed from the one or more electrodes on the lead system 108, thoracic or cardiac impedance signal, arterial pressure signal, pulmonary artery pressure signal, left atrial pressure signal, RV pressure signal, LV coronary pressure signal, coronary blood temperature signal, blood oxygen saturation signal, heart sound signal such as sensed by an ambulatory accelerometer or acoustic sensors, physiologic response to activity, apnea hypopnea index, one or more respiration signals such as a respiration rate signal or a tidal volume signal, brain natriuretic peptide (BNP), blood panel, sodium and potassium levels, glucose level and other biomarkers and bio-chemical markers, among others. In an example, the AMD 110 may record a physiologic signal when it detects a particular physiologic event, such as a cardiac arrhythmia episode, or a WHF event. In another example, the AMD 110 may record a physiologic signal in response to the patient trigger, also known as a patient-triggered episode. In yet another example, the AMD 110 may record a physiologic signal when it detects a syncope or a presyncope event.

The recorded physiologic signals may be stored in a device memory within the AMD 110, or in an external storage device such as an electronic medical record system. The data receiver 210 may retrieve from the device memory or the external storage device the stored physiologic signal. Data retrieval may be in response to a user command, or in response to a specific event. The received data may be processed by the data processed by the presentation control circuit 220 before being presented to a user.

The presentation control circuit 220 may be implemented as a part of a microprocessor circuit, which may be a dedicated processor such as a digital signal processor, application specific integrated circuit (ASIC), microprocessor, or other type of processor for processing information including physical activity information. Alternatively, the microprocessor circuit may be a general-purpose processor that may receive and execute a set of instructions of performing the functions, methods, or techniques described herein.

The presentation control circuit 220 includes circuit sets comprising one or more other circuits or sub-circuits, including a signal partitioner circuit 222, and optionally a signal metric circuit 224, and a data prioritizer circuit 240. These circuits may, alone or in combination, perform the functions, methods, or techniques described herein. In an example, hardware of the circuit set may be immutably designed to carry out a specific operation (e.g., hardwired). In an example, the hardware of the circuit set may include variably connected physical components (e.g., execution units, transistors, simple circuits, etc.) including a computer readable medium physically modified (e.g., magnetically, electrically, moveable placement of invariant massed particles, etc.) to encode instructions of the specific operation. In connecting the physical components, the underlying electrical properties of a hardware constituent are changed, for example, from an insulator to a conductor or vice versa. The instructions enable embedded hardware (e.g., the execution units or a loading mechanism) to create members of the circuit set in hardware via the variable connections to carry out portions of the specific operation when in operation. Accordingly, the computer readable medium is communicatively coupled to the other components of the circuit set member when the device is operating. In an example, any of the physical components may be used in more than one member of more than one circuit set. For example, under operation, execution units may be used in a first circuit of a first circuit set at one point in time and reused by a second circuit in the first circuit set, or by a third circuit in a second circuit set at a different time.

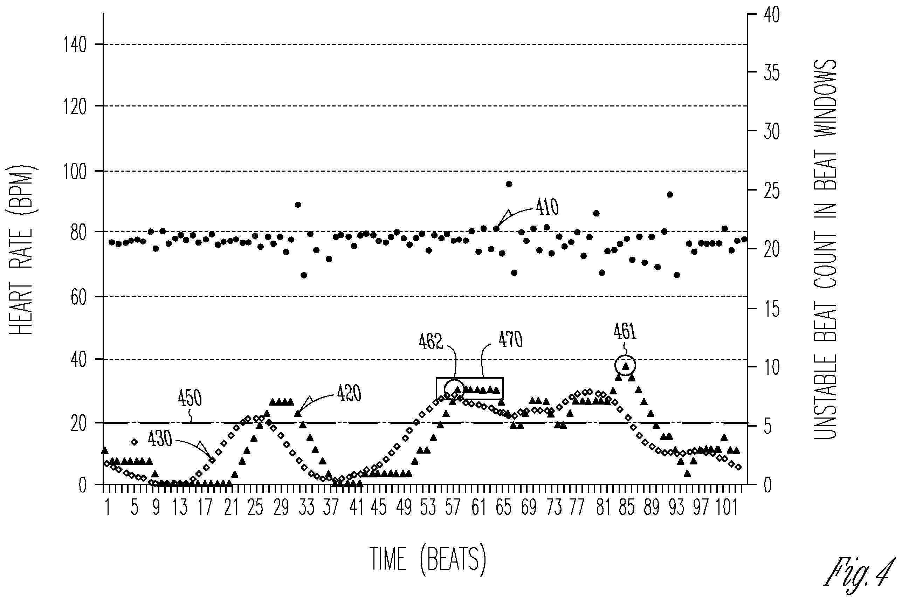

The signal partitioner circuit 222 partitions the physiologic data received from the sensor circuit 210 into a plurality of data subsets {X₁, X₂, ..., X_{N}}. The signal partitioner circuit 222 partitions a received physiologic signal, recorded during a device-detected cardiac event, into different signal portions. In an example, the signal portions are a plurality of beat windows each having a specific length, represented by specified number of heart beats or a time duration. The length of the beat windows may be programmable, such as adjustable by a user via the user interface 230. In an example, the beat window is approximately 2-5 minutes. In another example, the beat window has a length of 10-20 heart beats. The beat windows may be consecutive in time without overlapping to each other. In some examples, two or more beat windows may partly overlap each other. For example, the beat windows may be a rolling window of a fixed length (e.g., a fixed number of heart beats) that moves through the stored physiologic signal. Examples of the data partition and beat windows for scheduling presentation of various portions of a physiologic signal are discussed below, such as with reference to FIG. 4.

The signal metric circuit 224 may evaluate a signal metric for each of the data subsets produced by the signal partitioner circuit 222. The signal metric may represent a characteristic of the physiologic event associated with the physiologic signal. The signal metric may be dependent upon the type of physiologic event such as detected by the AMD 110. In an example of presenting physiologic data associated with an atrial tachyarrhythmia event such as AF, the signal metric circuit 224 may evaluate ventricular heart rate variability (HRV) over the data subsets taken from the physiologic data recorded during an AF episode. This is partly because AF typically has a variable ventricular heart rate. In another example of presenting physiologic data associated with a syncope or pre-syncope event, such as a cardiogenic syncope episode, the signal metric circuit 224 may evaluate a cardiac pause metric (e.g., a duration or a pattern of cardiac pause) over the data subsets taken from the physiologic data recorded during a detected syncope or pre-syncope episode. This is partly because a cardiogenic syncope typically has an extended cardiac pause. In another example, for a patient-triggered episode, the signal metric circuit 224 may evaluate one or more signal metrics for the data subsets including, for example, a heart rate metric, an ectopic beat metric such as a count of ectopic beats, a presence of a pause or an arrhythmia, an atrioventricular (AV) conduction metric such as presence of AV block, or a signal morphology metric, among others.

The data prioritizer circuit 226 may select a particular signal portion (a target subset or a target data window, X*) that satisfies a specific condition, such as more representative of a characteristic of the detected cardiac event than other subsets or signal portions. For example, in selecting a data subset from a physiologic signal corresponding to an AF episode, the selected target subset X* has an HRV value greater than other data subsets. The target subset or beat window X* thus selected is more representative of the characteristic of the detected episode. The data prioritizer circuit 226 may prioritize a presentation of the target subset or beat window X* over other data subsets to the user. This allows the user to more quickly and effectively recognize the arrhythmia type, or to adjudicate the detection decision made by the AMD 110 as either true positive detection (indicating the user's agreement with the device detection) or false positive detection (indicating the user's disagreement with the device detection). As such, the prioritized data presentation produced by the presentation control circuit 220 saves time, effort, and cost of data review and adjudication, and allows more timely intervention or adjustment of event detection such as by the AMD 110.

The user interface 230 may include an input device and an output device. In an example, at least a portion of the user interface 230 may be implemented in the external system 130. The input device may receive a user's programming input, such as parameters for determining and comparing the signal metrics over the multiple data subsets. The input device may include a keyboard, on-screen keyboard, mouse, trackball, touchpad, touch-screen, or other pointing or navigating devices. The input device may enable a system user to program the parameters used for sensing the physiologic signals, computing and signal metrics for the data subsets, and generating alerts, among others.

The output device may present the physiologic signal corresponding to a physiologic event to the user, such as displaying the physiologic signal on a display. The user may review the presented physiologic signal, and provide adjudication (e.g., an annotation of true positive or false positive detection) via the input device. In an example, the output device may present multiple data subsets (e.g., different signal portions or beat windows) according to the data prioritization as determined by the data prioritizer circuit 226. For example, in displaying a physiologic signal corresponding to a device-detected AF episode, the output device may display the target subset or beat window X* to the user prior to other data subsets. As discussed above, X* is a data portion that more likely to reveal the characteristic feature of AF, such as a large HRV, than other data subsets. By first presenting the target subset or beat window X*, the user may spend less time and effort reviewing the presented physiologic signal in order to make an adjudication decision or adjust device parameters for detecting a physiologic event or delivering a therapy. Compared to the conventional non-selective presentation of the stored physiologic data in its chronological order, the selective and prioritized data presentation discussed herein is a more efficient and cost-effective method of adjudication, particularly for users who hand a large volume of data for adjudication.

The output device may additionally present intermediate measurements or computations, such as values of signal metrics for the data subsets, to the user. The information may be presented in a table, a chart, a diagram, or any other types of textual, tabular, or graphical presentation formats. The presentation of the output information may include audio or other media format to alert the system user of the device-detected physiologic event associated with the physiological signal being displayed. The output unit may include a printer for printing hard copies of the detection information. In an example, the output device may generate alerts, alarms, emergency calls, or other forms of warnings to signal the system user about the detected arrhythmic events.

The optional therapy circuit 240 may be configured to deliver a therapy to the patient such as based on the user adjudication of the device-detected physiologic event. Examples of the therapy may include electrostimulation therapy delivered to the heart, a nerve tissue, other target tissues, a cardioversion therapy, a defibrillation therapy, or drug therapy. In some examples, the therapy circuit 240 may modify an existing therapy, such as adjust a stimulation parameter or drug dosage. For example, if there is a substantial amount of FP detections from the adjudication, one or more therapy parameters may be adjusted. The user may accept, reject, or modify the therapy adjustment such as via the input device.

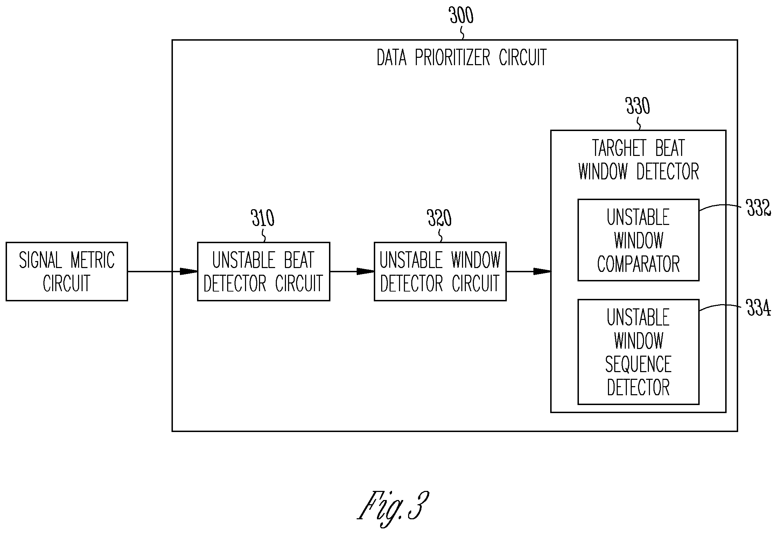

FIG. 3 illustrates an example of a data prioritizer circuit 300 configured to prioritize the data subsets taken from a physiologic signal corresponding to an AF episode as detected by the AMD 110. The data prioritizer circuit 300, which is an embodiment of the data prioritizer circuit 226, includes an unstable beat detector circuit 310, an unstable window detector circuit 320, and a target beat window detector 330.

The unstable beat detector circuit 310 may recognize a heart beat as either a stable beat or an unstable beat using a comparison of the cardiac cycle length (CL) of the present beat (CL(n)) and a reference CL. The CL may be represented by an R wave to R wave interval (RR interval), which can be measured from an ECG or an intracardiac electrogram. In an example, the stable or unstable beat can be recognized using a beat-to-beat change in cycle length, that is, ΔCL(n) = CL(n) - CL(n-1), where CL(n-1) is the cycle length immediately before the present heart beat. The unstable beat detector circuit 310 may recognize beat "n" as a stable beat if ΔCL(n) is less than the beat stability threshold, or as an unstable beat if ΔCL(n) is greater than the beat stability threshold. An exemplary beat stability threshold is approximately 5-10 beats per minute (bpm). In another example, the unstable beat detector circuit 310 may recognize beat "n" as a stable beat if ΔCL(n) falls within a first heart rate range, or as an unstable beat if ΔCL(n) falls into a second heart range. In an example, the first heart rate range is 0-5 bpm, and the second heart rate range is 5-100 bpm. In some examples, the unstable beat detector circuit 310 may recognize a beat as an unstable beat only if the heart rate is within a heart rate range. For example, the beat "n" is recognized as an unstable beat if ΔCL(n) falls within a specified range or exceeding a threshold, and that the heart rate around the beat "n", such as computed as average heart rate over 3-5 cardiac cycles around the beat "n" falls in a range of approximately 30-250 bpm.

The unstable window detector 320 is configured to detect one or more unstable beat windows from the plurality of beat windows, such as the data subsets provided by the signal partitioner circuit 222. Each beat window contains a specified number of heart beats. The unstable window detector 320 may recognize a beat window as an unstable window if there are a sufficient number of unstable beats (as recognized by the unstable beat detector circuit 310) in the beat window, such as exceeding an unstable beat count threshold, expressed as an absolute count number or a relative number (e.g., a fraction or percentage). In an example, each beat window includes 10 ventricular heart beats, and a beat window is determined as an unstable window if it includes at least 50%, or five or more, unstable beats.

In some examples, the unstable window detector 320 may trend the count of unstable heart beats over the beat windows, and smoothen the trend such as by using a low-pass filter or computing a moving average of the unstable heart beat counts. Based on the smoothened trend, the unstable window detector 320 may re-determine the count of unstable heart beats in each of the beat windows, and recognize an unstable window if the re-determined count of unstable heart beat contained therein exceeds an unstable beat count threshold.

If the unstable window detector circuit 320 recognizes only one unstable window out of the plurality of data windows being examined, then the target beat window detector 330 may determine the recognized unstable window as the target beat window X*, which may then be prioritized for presentation to a user, such as via the user interface 230, prior to other beat windows or subsets. However, if the unstable window detector circuit 320 recognized two or more unstable windows from the plurality of data windows being examined, then the target beat window detector 330 may select at least one target beat window target subset X* using one of the unstable window comparator 332, or an unstable window sequence detector 334. In an example, the unstable window comparator 332 may determine a target beat window X* based on timing of the recognized unstable windows. In an example, the target beat window X* may be determined to be the unstable window first detected in time. In an example, once the first unstable window is detected, that unstable window is determined as the target beat window X*; and the unstable window detector circuit 320 may stop the unstable window assessment on the remainder of the beat windows. In another example, the unstable window comparator 332 determine a target beat window X* based on the number of unstable beats in the recognized unstable windows. In an example, the unstable window comparator 332 may compare the number of unstable beats of the recognized unstable windows, and determine a target beat window X* to be the unstable window that has the most unstable heart beats among the beat windows being examined. In yet another example unstable window comparator 332 may recognize the target beat window X* based on a pattern of the unstable beats in the recognized unstable windows. An example of the unstable beat pattern includes a beat sequence of a consecutive number of unstable beats. In an example, the unstable window comparator 332 may determine a target beat window X* to be the unstable window that has a sequence of consecutive unstable beats exceeding a threshold number of unstable beats, or that has the longest sequence of consecutive unstable beats among the beat windows being examined.

The unstable window sequence detector 334 may recognize the target beat window X* based on a pattern of the two or more recognized beat windows. In an example, the unstable window sequence detector 334 may detect a sequence of consecutive unstable windows {W₁, W₂, ..., Wₖ}, such that no stable window (i.e., a beat window that does not satisfy the unstable beat requirement as set in the unstable window detector circuit 320) is detected between any two adjacent unstable windows Wᵢ and Wᵢ₊₁. If the detected consecutive unstable window sequence is sufficiently long, such that the length of the sequence, k, exceeding a threshold of unstable window count, then the unstable window sequence detector 334 may determine the unstable window that leads said unstable window sequence, which is W₁ in this example, as the target beat window X*. Alternatively, when two or more sequences of consecutive unstable windows are detected (particularly if a small threshold, e.g., 3-5, of unstable window count is used), the unstable window sequence detector 334 may identify the longest sequence of consecutive unstable windows, and determine the unstable window that leads the longest unstable window sequence to be the target beat window X*. Examples of determining the target beat window X* using the unstable beats in a recognized unstable window, or the unstable window sequence, are discussed below, such as with reference to FIG. 4.

FIG. 4 is a graph illustrating an example of selecting a portion of a heart rate time series 410 for presenting to a user (e.g., a clinician) to adjudicate a detection of an AF episode, such as detected by the AMD 110 from a patient. The graph may be presented to a user via the user interface 230, such as displayed on a display unit. The heart rate time series 410 represents beat-to-beat ventricular heart rates (beats per minute, or bpm, in the left-side y-axis) over time (beat indices in the x-axis). The sensor circuit 210 may sense an ECG or a ventricular electrogram (EGM) signal such as recorded by the AMD 110 in response to the AF detection, detect ventricular activation, or heart beats, from the ECG or the EGM, measure beat-to-beat cardiac cycle lengths, and determine beat-to-beat heart rates. As illustrated in FIG.4, the portion of the heart rate time series 410 as shown has an average rate of approximately 75-80 bpm, and fluctuates over time, with a more variable heart rate in the latter portion (e.g., heart beats after beat index 60 or so) than the earlier portion (e.g., heart beats before beat index 60 or so).

Also shown in FIG. 4 is an unstable beat count trend 420, representing the number of unstable heart beats of a plurality of beat windows (in the right-side y-axis). The beat windows each capture a subset of heart rate time series 410. The beat windows may partly overlap. By way of example and not limitation, the beat window contains 10 heart beats, moves through the heart rate time series 410 with 90% (i.e., 9 heart beats) overlapping with its adjacent beat window. Unstable beats may be detected from each beat window, and the number of unstable beats may be determined, such as using the unstable beat detector circuit 310. Unstable beat windows may be recognized as those beat windows with the unstable beat count exceeding a threshold 450 (which is five in the example of FIG. 4), such as using the unstable window detector circuit 320.

A target beat window X* may then be determined from the recognized unstable windows. In an example, the unstable window comparator 332 may be used to select a target beat window X* corresponding to the unstable beat count 461, which is the largest of the unstable beat counts over the recognized unstable windows as illustrated in the unstable beat count trend 420. The target beat window X* thus selected has the most unstable heart beats among the beat windows being examined.

Alternatively, the unstable window sequence detector 334 may be used to select a target beat window X* corresponding to the unstable beat count 462, which leads a sequence 470 of consecutive unstable windows each having the required number of unstable beats, such as 8 or more unstable beats within each beat window. If two or more sequences of consecutive unstable windows are detected, then the unstable window that leads the longest unstable window sequence is determined to be the target beat window X*. The portion of the recorded ECG or EGM within the target beat window X* may then be presented to the user with a priority higher than other portions or beat windows of the recorded ECG or EGM.

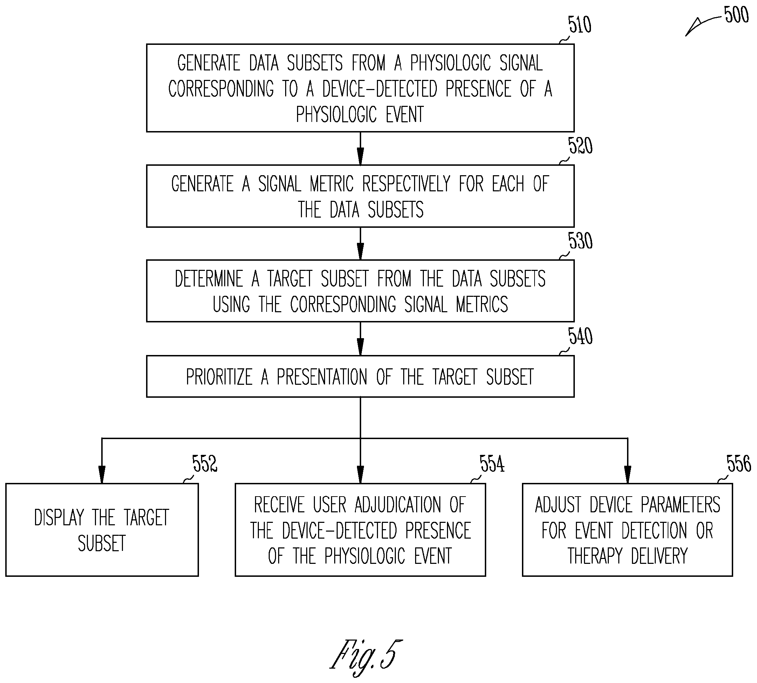

FIG. 5 illustrates generally an example of a method 500 for presenting physiologic data to a user on a user interface. The physiologic data may include a physiologic signal recorded by a medical device, such as the AMD 110, in response to a device-detected presence of a physiologic event, such as a cardiac arrhythmia episode, a worsening heart failure (WHF) event, a syncope or a presyncope event, or a patient-triggered event. The method 500 may be implemented in and executed by the external system 125, distributedly between the external system 125 and the AMD 110, or the data presentation system 200 as previously discussed.

The method 500 commences at step 510, where data subsets may be generated using a physiologic signal corresponding to a device-detected presence of a physiologic event. Examples of the physiological signals may include surface electrocardiography (ECG), subcutaneous ECG, intracardiac electrogram (EGM), thoracic or cardiac impedance signal, blood pressure signal, blood oxygen saturation signal, heart sound signal, physiological response to activity, apnea hypopnea index, one or more respiration signals such as a respiration rate signal or a tidal volume signal, brain natriuretic peptide (BNP), blood panel, sodium and potassium levels, glucose level and other biomarkers and bio-chemical markers, among others. The physiologic signal may be partitioned into a plurality of data subsets{X₁, X₂, ..., X_{N}}, such as by using the signal partition circuit 222. In an example, the beat windows may be generated using a rolling window of a specified window length (e.g. a specified number of heart beats) moving through the physiological signal. The beat windows may be consecutive without overlapping, or at least partly overlap each other.

At 520, a signal metric may be generated for the data subsets or beat windows, such as by using the signal metric circuit 224. The signal metric may be dependent upon the type of device-detected physiological event. For example, for a ECG or EGM recorded in response to a device-detected atrial tachyarrhythmia episode, ventricular heart rate variability (HRV) metric may be generated for the beat windows created from the ECG or ECG signal corresponding to the detected atrial tachyarrhythmia episode. In another example, for a physiologic signal recorded in response to a patient-triggered episode, one or more metrics may be generated for the beat windows, including a heart rate metric, an ectopic beat metric such as a count of ectopic beats, a presence of a pause or an arrhythmia, an atrioventricular (AV) conduction metric such as presence of AV block, or a signal morphology metric, among others. In yet another example, for a physiologic signal recorded in response to a device-detected syncope or pre-syncope event, a cardiac pause metric (e.g., a duration or a pattern of cardiac pause) may be generated for the beat windows.

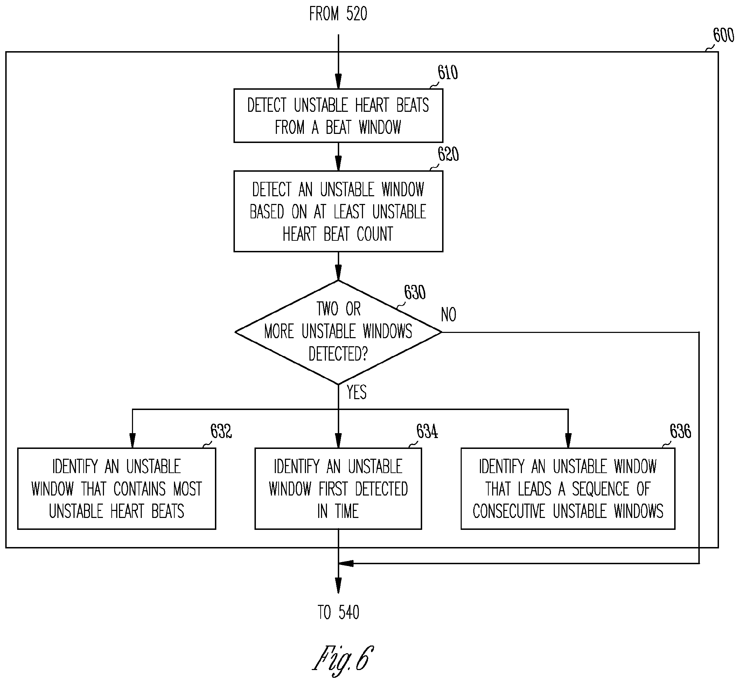

At 530, a target subset may be selected from the multiple data subsets using the signal metrics respectively generated for data subsets. The target subset may be selected such that the corresponding signal metric generated therefrom may be more representative of a characteristic of the device-detected physiologic event than other data subsets or beat windows. In an example of presenting a device-detected AF episode, a target beat window may be selected as one of the multiple beat windows that has a heart rate variability (HRV) value greater than other data subsets. The target subset thus selected is more representative of the characteristic of the device-detected AF episode. At 540, the data prioritizer circuit 226 may prioritize a presentation of the target subset or beat window X* over other data subsets to the user. Determination of the target subset at 530 and prioritized presentation of the target subset at 540 may be executed using the data prioritizer circuit 226. Examples of selecting the target subset or a target beat window are discussed below, such as with reference to FIG. 6.

The target subset of the physiologic signal may be provided to one or more processes 552, 554, or 556. At 552, the physiologic signal may be output to a user according to the prioritization of the data subsets as determined at 540. In an example, the target subset of the physiologic signal may be displayed on a display of the user interface 230. Other data subsets of the physiologic signal with lower priorities than the target subset may be displayed after the target subset has been displayed, such as via a user command via a user input of the user interface 230. In various examples, signal metrics of the target subset and other data subsets may also be presented to the user, such as displayed on a display unit or printed in a hard copy. In various examples, alerts, alarms, emergency calls, or other forms of warnings may be generated to inform the user about the device-detected physiologic event associated with the physiological signal being displayed.

At 554, a user adjudication of the device-detected presence of the physiologic event (e.g., a detection of a cardiac arrhythmia episode generated by the AMD 110) may be received. The adjudication may include an indication that the detection as made by the device (e.g., the AMD 110) is either a true positive detection (indicating the user's agreement with the device detection) or a false positive detection (indicating the user's disagreement with the device detection). Because the target subset may more likely reveal the characteristics of the device-detected physiologic event than other data subsets, the prioritized presentation of the target subset or beat window may allow the user to more quickly and effectively recognize a presence or absence of a physiologic event, thereby saving the user's time and effort in reviewing the physiologic signal in order to make an adjudication decision.

At 556, one or more device parameters for physiologic event detection or therapy delivery may be adjusted, such as based on the user adjudication of the device-detected presence of the physiologic event. For example, if there is a substantial amount of false positive detections from the adjudication, one or more therapy parameters may be adjusted. The user may accept, reject, or modify the therapy adjustment such as via the user interface 230. The parameters may be adjusted automatically or manually by the user. In an example, a recommendation may be generated and provided to a user, including further diagnostic tests to be performed, implantation of a device, adjustment of one or more physiologic event detection parameters or therapy parameters (e.g., electrostimulation parameters or drug dosage). Therapy may be delivered according to the adjusted therapy parameters. Examples of the therapy may include electrostimulation therapy delivered to the heart, a nerve tissue, other target tissues, a cardioversion therapy, a defibrillation therapy, or drug therapy.

FIG. 6 is a flowchart illustrating an example of a method 600 for prioritizing the data subsets taken from a physiologic signal corresponding to a device-detected AF episode. The method 600 may be an embodiment of step 530 of determining the target subset as illustrated in FIG. 5. The method 600 begins at 610 to detect unstable heart beats from each of a plurality of beat windows, such as by using the unstable beat detector circuit 310. The beat windows are data subsets taken from an ECG or an EGM recorded in response to a device-detected presence of an AF episode. In an example, each beat window includes 10-20 ventricular heart beats. Unstable beats can be detected using a beat-to-beat change in cycle length (e.g., R wave to R wave interval in an ECG or an EGM), denoted as ΔCL(n) at a heart beat "n". If a difference between the cycle length at a present heart beat and the cycle length immediately before the present heart beat exceeds a threshold value, or falls within a specified range, then the heart beat is detected as an unstable beat. In some examples, recognition of an unstable beat may further require that the heart rate be within a heart rate range, such as between approximately 30-250 bpm.

At 620, an unstable window may be detected based on at least a count of unstable heart beats in a beat window. A beat window may be recognized as an unstable window if there are a sufficient number of unstable beats in the beat window, such as exceeding an unstable beat count threshold. In an example, a beat window is deemed unstable if 50% or more of the heart beats therein are unstable beats.

At 630, the unstable windows detected at step 620 are analyzed to identify at least one target beat window. If at 630 only one unstable window is detected from the plurality of data windows being examined, then the only detected unstable window may be recognized as the target beat window, and the presentation of such a target beat window may be prioritized at step 540 of FIG. 5. If at 630 two or more unstable windows are detected from the data windows being examined, then one of alternative methods 632, 634, or 636 may be executed to determine the target beat window.

At 632, a target beat window may be detected based on the number of unstable beats in the recognized unstable windows. An unstable window that has the most (i.e., the largest number of) unstable heart beats may be identified, and determined to be the target beat window. At 634, a target beat window may be detected based on timing of the recognized unstable windows. An unstable window that is first detected in time may be identified, and determined to be the target beat window. At 636, a target beat window may be detected based on a pattern of two or more recognized beat windows. A sequence of consecutive unstable windows {W₁, W₂, ..., Wₖ} may be identified, such as using the unstable window sequence detector 334. If the detected consecutive unstable window sequence is sufficiently long, such that the length of the sequence, k, exceeding a threshold of unstable window count, then the unstable window that leads said unstable window sequence may be determined to be the target beat window. Alternatively, when two or more sequences of consecutive unstable windows are detected, the longest sequence of consecutive unstable windows may be identified, and the unstable window that leads the longest unstable window sequence may be determined to be the target beat window. The target beat window determined at one of the steps 632, 634, or 636 may be provided to step 540 to prioritize beat window presentation to the user.

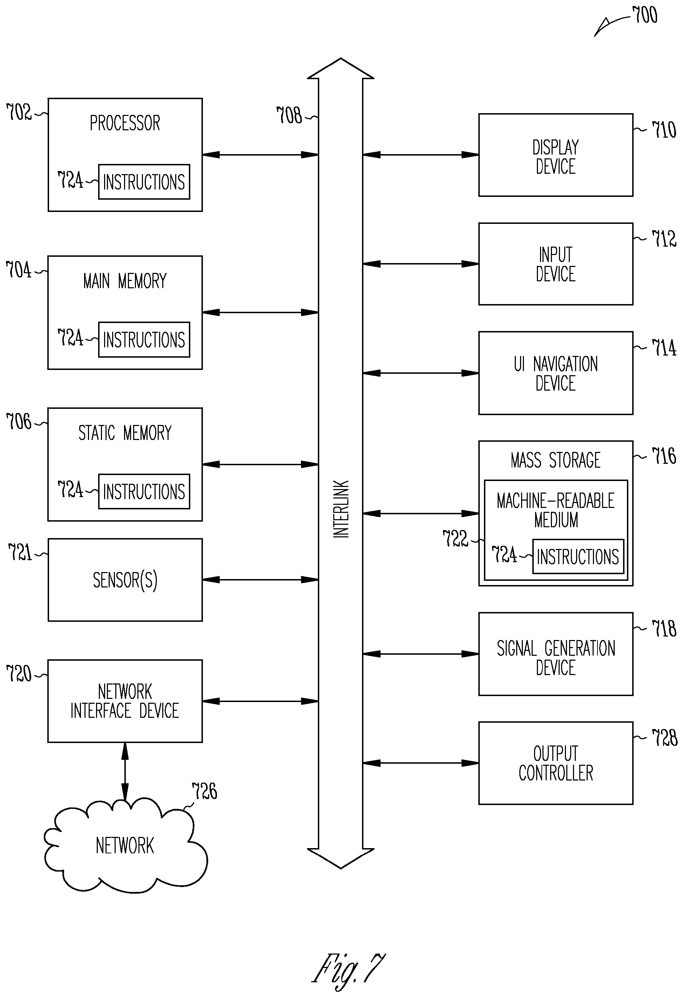

FIG. 7 illustrates generally a block diagram of an example machine 700 upon which any one or more of the techniques (e.g., methodologies) discussed herein may perform. Portions of this description may apply to the computing framework of various portions of the LCP device, the IMD, or the external programmer.

In alternative embodiments, the machine 700 may operate as a standalone device or may be connected (e.g., networked) to other machines. In a networked deployment, the machine 700 may operate in the capacity of a server machine, a client machine, or both in server-client network environments. In an example, the machine 700 may act as a peer machine in peer-to-peer (P2P) (or other distributed) network environment. The machine 700 may be a personal computer (PC), a tablet PC, a set-top box (STB), a personal digital assistant (PDA), a mobile telephone, a web appliance, a network router, switch or bridge, or any machine capable of executing instructions (sequential or otherwise) that specify actions to be taken by that machine. Further, while only a single machine is illustrated, the term "machine" shall also be taken to include any collection of machines that individually or jointly execute a set (or multiple sets) of instructions to perform any one or more of the methodologies discussed herein, such as cloud computing, software as a service (SaaS), other computer cluster configurations.

Examples, as described herein, may include, or may operate by, logic or a number of components, or mechanisms. Circuit sets are a collection of circuits implemented in tangible entities that include hardware (e.g., simple circuits, gates, logic, etc.). Circuit set membership may be flexible over time and underlying hardware variability. Circuit sets include members that may, alone or in combination, perform specified operations when operating. In an example, hardware of the circuit set may be immutably designed to carry out a specific operation (e.g., hardwired). In an example, the hardware of the circuit set may include variably connected physical components (e.g., execution units, transistors, simple circuits, etc.) including a computer readable medium physically modified (e.g., magnetically, electrically, moveable placement of invariant massed particles, etc.) to encode instructions of the specific operation. In connecting the physical components, the underlying electrical properties of a hardware constituent are changed, for example, from an insulator to a conductor or vice versa. The instructions enable embedded hardware (e.g., the execution units or a loading mechanism) to create members of the circuit set in hardware via the variable connections to carry out portions of the specific operation when in operation. Accordingly, the computer readable medium is communicatively coupled to the other components of the circuit set member when the device is operating. In an example, any of the physical components may be used in more than one member of more than one circuit set. For example, under operation, execution units may be used in a first circuit of a first circuit set at one point in time and reused by a second circuit in the first circuit set, or by a third circuit in a second circuit set at a different time.

Machine (e.g., computer system) 700 may include a hardware processor 702 (e.g., a central processing unit (CPU), a graphics processing unit (GPU), a hardware processor core, or any combination thereof), a main memory 704 and a static memory 706, some or all of which may communicate with each other via an interlink (e.g., bus) 708. The machine 700 may further include a display unit 710 (e.g., a raster display, vector display, holographic display, etc.), an alphanumeric input device 712 (e.g., a keyboard), and a user interface (UI) navigation device 714 (e.g., a mouse). In an example, the display unit 710, input device 712 and UI navigation device 714 may be a touch screen display. The machine 700 may additionally include a storage device (e.g., drive unit) 716, a signal generation device 718 (e.g., a speaker), a network interface device 720, and one or more sensors 721, such as a global positioning system (GPS) sensor, compass, accelerometer, or other sensors. The machine 700 may include an output controller 728, such as a serial (e.g., universal serial bus (USB), parallel, or other wired or wireless (e.g., infrared (IR), near field communication (NFC), etc.) connection to communicate or control one or more peripheral devices (e.g., a printer, card reader, etc.).

The storage device 716 may include a machine readable medium 722 on which is stored one or more sets of data structures or instructions 724 (e.g., software) embodying or utilized by any one or more of the techniques or functions described herein. The instructions 724 may also reside, completely or at least partially, within the main memory 704, within static memory 706, or within the hardware processor 702 during execution thereof by the machine 700. In an example, one or any combination of the hardware processor 702, the main memory 704, the static memory 706, or the storage device 716 may constitute machine-readable media.

While the machine-readable medium 722 is illustrated as a single medium, the term "machine readable medium" may include a single medium or multiple media (e.g., a centralized or distributed database, and/or associated caches and servers) configured to store the one or more instructions 724.

The term "machine readable medium" may include any medium that is capable of storing, encoding, or carrying instructions for execution by the machine 700 and that cause the machine 700 to perform any one or more of the techniques of the present disclosure, or that is capable of storing, encoding or carrying data structures used by or associated with such instructions. Nonlimiting machine-readable medium examples may include solid-state memories, and optical and magnetic media. In an example, a massed machine-readable medium comprises a machine readable medium with a plurality of particles having invariant (e.g., rest) mass. Accordingly, massed machine-readable media are not transitory propagating signals. Specific examples of massed machine-readable media may include: non-volatile memory, such as semiconductor memory devices (e.g., Electrically Programmable Read-Only Memory (EPROM), Electrically Erasable Programmable Read-Only Memory (EEPROM)) and flash memory devices; magnetic disks, such as internal hard disks and removable disks; magneto-optical disks; and CD-ROM and DVD-ROM disks.

The instructions 724 may further be transmitted or received over a communications network 726 using a transmission medium via the network interface device 720 utilizing any one of a number of transfer protocols (e.g., frame relay, internet protocol (IP), transmission control protocol (TCP), user datagram protocol (UDP), hypertext transfer protocol (HTTP), etc. ). Example communication networks may include a local area network (LAN), a wide area network (WAN), a packet data network (e.g., the Internet), mobile telephone networks (e.g., cellular networks), Plain Old Telephone (POTS) networks, and wireless data networks (e.g., Institute of Electrical and Electronics Engineers (IEEE) 802. 11 family of standards known as WiFi^{®}, IEEE 802. 16 family of standards known as WiMax^{®}), IEEE 802. 15. 4 family of standards, peer-to-peer (P2P) networks, among others. In an example, the network interface device 720 may include one or more physical jacks (e.g., Ethernet, coaxial, or phone jacks) or one or more antennas to connect to the communications network 726. In an example, the network interface device 720 may include a plurality of antennas to wirelessly communicate using at least one of single-input multiple-output (SIMO), multiple-input multiple-output (MIMO), or multiple-input single-output (MISO) techniques. The term "transmission medium" shall be taken to include any intangible medium that is capable of storing, encoding or carrying instructions for execution by the machine 700, and includes digital or analog communications signals or other intangible medium to facilitate communication of such software.

Various embodiments are illustrated in the figures above. One or more features from one or more of these embodiments may be combined to form other embodiments.

The method examples described herein can be machine or computer-implemented at least in part. Some examples may include a computer-readable medium or machine-readable medium encoded with instructions operable to configure an electronic device or system to perform methods as described in the above examples. An implementation of such methods may include code, such as microcode, assembly language code, a higher-level language code, or the like. Such code may include computer readable instructions for performing various methods. The code can form portions of computer program products. Further, the code can be tangibly stored on one or more volatile or non-volatile computer-readable media during execution or at other times.

The above detailed description is intended to be illustrative, and not restrictive. The scope of the invention is determined by the appended claims.

## Claims

1. A system (200) for presenting physiologic data to a user, comprising:
a presentation control circuit (220) configured to:
generate a signal metric respectively for data subsets of a physiologic signal corresponding to a device-detected presence of a physiologic event, the signal metric representing a characteristic of the physiologic event;
determine, from the data subsets of the physiologic signal, a target subset with the corresponding generated signal metric satisfying a specific condition; and
present the determined target subset of the physiologic signal to the user,
**characterised in that**
the presentation control circuit (220) includes a signal partitioner circuit (222) configured to generate the data subsets by partitioning a physiological signal, recorded during a device-detected cardiac event, into different signal portions.

2. The system (200) of claim 1, comprising a user interface (230) configured to:
display the target subset prior to other data subsets of the physiologic signal; and
receive a user adjudication of the device-detected presence of the physiologic event.

3. The system (200) of any of claims 1-2, wherein the physiologic signal is recorded in response to a device-detected presence of a cardiac arrhythmia episode, and the signal metric represents a cardiac timing or a signal morphology of the recorded physiologic signal.

4. The system (200) of any of claims 3, wherein the physiologic signal is recorded in response to a device-detected presence of an atrial tachyarrhythmia episode, and the signal metric includes a ventricular heart rate variability (HRV).

5. The system (200) of any of claims 1-2, wherein the physiologic signal is recorded in response to a device-detected presence of a syncope or a presyncope, and the signal metric includes a cardiac pause metric.

6. The system (200) of claim 4, wherein the presentation control circuit (220) is configured to:
detect an unstable heart beat from each of the data subsets of the physiologic signal based on the HRV;
determine, for the data subsets, respective counts of the detected unstable heart beat; and
determine the target subset using the determined counts of unstable heart beats for the data subsets.

7. The system (200) of claim 6, wherein the presentation control circuit (220) is configured to detect the unstable heart beat when a beat-to-beat change in ventricular heart rate exceeds a beat stability threshold, or falls within a beat stability range.

8. The system (200) of claim 7, wherein the presentation control circuit (220) is configured to detect the unstable heart beats further requiring that a heart rate of the data subset is within a heart rate range.

9. The system (200) of claim 6, wherein the data subsets of the physiologic signal include beat windows including a specified number of heart beats, and the presentation control circuit (220) is configured to:
detect, from the beat windows, one or more unstable windows with corresponding counts of unstable heart beats exceeding a unstable beat count threshold; and
determine the target subset using the detected one or more unstable windows.

10. The system (200) of claim 9, wherein the presentation control circuit (220) is configured to determine the target subset corresponding to an unstable window containing a maximum count of unstable heart beats among the unstable beat windows.

11. The system (200) of any of claims 9-10, wherein the presentation control circuit (220) is configured to determine the target subset corresponding to an unstable window first detected in time among the unstable beat windows.

12. The system (200) of any of claims 9-11, wherein the presentation control circuit (220) is configured to determine the target subset corresponding to an unstable window that leads a sequence of consecutive unstable windows exceeding a threshold number of unstable windows.

13. The system (200) of claim 12, wherein the presentation control circuit (220) is configured to detect, from two or more sequences of consecutive unstable windows, a longest consecutive unstable window sequence, and to determine the target subset corresponding to an unstable window that leads the longest sequence of consecutive unstable windows.

14. The system (200) of any of claims 9-13, wherein the presentation control circuit (220) is further configured to:
smoothen a trend of unstable heart beat counts over the beat windows; and
detect one or more unstable windows each having the corresponding unstable heart beat count, determined from the smoothened trend, that exceeds the unstable beat count threshold.

15. The system (200) of claim 14, wherein the presentation control circuit (220) is configured to smoothen the trend of the count of unstable heart beats using a moving average of the trend of unstable heart beat counts over the beat windows.

## Patentansprüche

1. System (200) zum Darstellen physiologischer Daten zu einem Benutzer, umfassend:
eine Darstellungssteuerschaltung (220), die eingerichtet ist zum:
Erzeugen einer Signalmetrik für jeweils Datenteilmengen eines physiologischen Signals, entsprechend einem von einer Vorrichtung erkannten Vorliegen eines physiologischen Ereignisses, wobei die Signalmetrik eine Eigenschaft des physiologischen Ereignisses darstellt;
Bestimmen aus den Datenteilmengen des physiologischen Signals einer Zielteilmenge mit der entsprechenden erzeugten Signalmetrik, die eine bestimmte Bedingung erfüllt; und
Darstellen zum Benutzer der erfassten Zielteilmenge des physiologischen Signals,
**dadurch gekennzeichnet, dass** die Darstellungssteuerschaltung (220) eine Signalpartitionierschaltung (222) umfasst, die eingerichtet ist, die Datenteilmengen zu erzeugen, indem sie ein physiologisches Signal, das während eines von der Vorrichtung erkannten Herzereignisses aufgezeichnet wird, in verschiedene Signalteile aufteilt.

2. System (200) nach Anspruch 1, das eine Benutzerschnittstelle (230) umfasst, eingerichtet zum:
Anzeigen der Zielteilmenge vor anderen Datenteilmengen des physiologischen Signals; und
Empfangen einer Benutzerbeurteilung des von der Vorrichtung erkannten Vorliegens des physiologischen Ereignisses.

3. System (200) gemäß einem der Ansprüche 1-2, wobei das physiologische Signal als Reaktion auf eine von einer Vorrichtung erkannte Herzrhythmusstörung aufgezeichnet wird und die Signalmetrik einen kardialen Zeitparameter oder eine Signalmorphologie des aufgezeichneten physiologischen Signals darstellt.

4. System (200) gemäß einem der Ansprüche 3, wobei das physiologische Signal als Reaktion auf eine von einer Vorrichtung erkannte Vorhoftachyarrhythmie-Episode aufgezeichnet wird und die Signalmetrik eine ventrikuläre Herzfrequenzvariabilität (HRV) umfasst.

5. System (200) gemäß einem der Ansprüche 1-2, wobei das physiologische Signal als Reaktion auf eine von einer Vorrichtung erkannte Synkope oder Präsynkope aufgezeichnet wird und die Signalmetrik eine Herzpausenmetrik umfasst.

6. System (200) nach Anspruch 4, wobei die Darstellungssteuerschaltung (220) eingerichtet ist zum:
Erkennen eines instabilen Herzschlags aus jeder der Datenteilmengen des physiologischen Signals auf der Grundlage der HRV;
Bestimmen, für die Datenteilmengen, der jeweiligen Anzahlen der erkannten instabilen Herzschläge; und
Bestimmen der Zielteilmenge anhand der bestimmten Anzahl instabiler Herzschläge für die Datenteilmengen.

7. System (200) gemäß Anspruch 6, wobei die
Darstellungssteuerschaltung (220) eingerichtet ist, den instabilen Herzschlag zu erkennen, wenn eine Schlag-zu-Schlag-Änderung der ventrikulären Herzfrequenz einen Schlagstabilitätsschwellenwert überschreitet oder in einen Schlagstabilitätsbereich fällt.

8. System (200) nach Anspruch 7, wobei die Darstellungssteuerschaltung (220) eingerichtet ist, die instabilen Herzschläge zu erkennen, wobei zusätzlich erforderlich ist, dass eine Herzfrequenz der Datenteilmenge innerhalb eines Herzfrequenzbereichs liegt.

9. System (200) nach Anspruch 6, wobei die Datenteilmengen des physiologischen Signals Schlagfenster mit einer bestimmten Anzahl von Herzschlägen umfassen und die Darstellungssteuerschaltung (220) eingerichtet ist zum:
Erkennen eines oder mehrerer instabiler Fenster aus den Herzschlagfenstern, deren entsprechende Anzahl instabiler Herzschläge einen Schwellenwert für instabile Herzschläge überschreitet; und
Bestimmen der Zielteilmenge anhand des/der erkannten instabilen Fensters/Fenster.

10. System (200) nach Anspruch 9, wobei die Darstellungssteuerschaltung (220) eingerichtet ist, die Zielteilmenge zu bestimmen, die einem instabilen Fenster entspricht, das eine maximale Anzahl instabiler Herzschläge unter den instabilen Schlagfenstern enthält.

11. System (200) nach einem der Ansprüche 9 bis 10, wobei die Darstellungssteuerschaltung (220) eingerichtet ist, die Zielteilmenge zu bestimmen, die einem instabilen Fenster entspricht, das unter den instabilen Herzschlagfenstern zeitlich zuerst erkannt wird.

12. System (200) nach einem der Ansprüche 9 bis 11, wobei die Darstellungssteuerschaltung (220) eingerichtet ist, die Zielteilmenge zu bestimmen, die einem instabilen Fenster entspricht, das eine Folge aufeinanderfolgender instabiler Fenster anführt, die eine Schwellenanzahl instabiler Fenster überschreiten.

13. System (200) nach Anspruch 12, wobei die
Darstellungssteuerschaltung (220) eingerichtet ist, aus zwei oder mehr Sequenzen aufeinanderfolgender instabiler Fenster eine längste Sequenz aufeinanderfolgender instabiler Fenster zu erkennen und die Zielteilmenge zu bestimmen, die einem instabilen Fenster entspricht, das die längste Sequenz aufeinanderfolgender instabiler Fenster anführt.

14. System (200) nach einem der Ansprüche 9-13, wobei die Darstellungssteuerschaltung (220) ferner eingerichtet ist zum:
Glätten eines Trends instabiler Herzschlagzahlen über die Schlagfenster; und
Erkennen eines oder mehrerer instabiler Fenster, die jeweils die entsprechende instabile Herzschlagzahl aufweisen, die aus dem geglätteten Trend bestimmt wurde und den Schwellenwert für die instabile Herzschlagzahl überschreitet.

15. System (200) nach Anspruch 14, wobei die Darstellungssteuerschaltung (220) eingerichtet ist, den Trend der Anzahl instabiler Herzschläge unter Verwendung eines gleitenden Mittelwerts des Trends der Anzahl instabiler Herzschläge über die Schlagfenster zu glätten.

## Revendications

1. Système (200) pour présenter des données physiologiques à un utilisateur, comprenant :
un circuit de commande de présentation (220) configuré pour :
générer une mesure de signal respectivement pour des sous-ensembles de données d'un signal physiologique correspondant à une présence détectée par dispositif d'un événement physiologique, la mesure de signal représentant une caractéristique de l'événement physiologique ;
déterminer, à partir des sous-ensembles de données du signal physiologique, un sous-ensemble cible dont la mesure de signal générée correspondante satisfait une condition spécifique ; et
présenter le sous-ensemble cible déterminé du signal physiologique à l'utilisateur,
**caractérisé en ce que** le circuit de commande de présentation (220) inclut un circuit de partitionnement de signal (222) configuré pour générer les sous-ensembles de données en partitionnant un signal physiologique, enregistré pendant un événement cardiaque détecté par dispositif, selon différentes parties de signal.

2. Système (200) selon la revendication 1, comprenant une interface utilisateur (230) configurée pour :
afficher le sous-ensemble cible avant d'autres sous-ensembles de données du signal physiologique ; et
recevoir une décision d'arbitrage d'utilisateur quant à la présence détectée par dispositif de l'événement physiologique.

3. Système (200) selon l'une quelconque des revendications 1 et 2, dans lequel le signal physiologique est enregistré en réponse à une présence détectée par dispositif d'un épisode d'arythmie cardiaque, et la mesure de signal représente une temporisation cardiaque ou une morphologie de signal du signal physiologique enregistré.

4. Système (200) selon l'une quelconque des revendications 3, dans lequel le signal physiologique est enregistré en réponse à une présence détectée par dispositif d'un épisode de tachyarythmie auriculaire, et la mesure de signal inclut une variabilité de rythme cardiaque ventriculaire (HRV).

5. Système (200) selon l'une quelconque des revendications 1 et 2, dans lequel le signal physiologique est enregistré en réponse à une présence détectée par dispositif d'une syncope ou d'une pré-syncope, et la mesure de signal inclut une mesure de pause cardiaque.

6. Système (200) selon la revendication 4, dans lequel le circuit de commande de présentation (220) est configuré pour :
détecter un battement cardiaque instable à partir de chacun des sous-ensembles de données du signal physiologique sur la base de la HRV;
déterminer, pour les sous-ensembles de données, des comptages respectifs du battement cardiaque instable détecté ; et
déterminer le sous-ensemble cible en utilisant les comptages déterminés de battements cardiaques instables pour les sous-ensembles de données.

7. Système (200) selon la revendication 6, dans lequel le circuit de commande de présentation (220) est configuré pour détecter le battement cardiaque instable lorsqu'une variation battement à battement du rythme cardiaque ventriculaire excède un seuil de stabilité de battement, ou tombe à l'intérieur d'une plage de stabilité de battement.

8. Système (200) selon la revendication 7, dans lequel le circuit de commande de présentation (220) est configuré pour détecter les battements cardiaques instables en imposant en outre qu'un rythme cardiaque du sous-ensemble de données soit à l'intérieur d'une plage de rythmes cardiaques.

9. Système (200) selon la revendication 6, dans lequel les sous-ensembles de données du signal physiologique incluent des fenêtres de battements qui incluent un nombre spécifié de battements cardiaques, et le circuit de commande de présentation (220) est configuré pour :
détecter, parmi les fenêtres de battements, une ou plusieurs fenêtres instables dont des comptages correspondants de battements cardiaques instables excèdent un seuil de comptage de battements instables ; et
déterminer le sous-ensemble cible en utilisant les une ou plusieurs fenêtres instables détectées.

10. Système (200) selon la revendication 9, dans lequel le circuit de commande de présentation (220) est configuré pour déterminer le sous-ensemble cible qui correspond à une fenêtre instable qui contient un comptage maximum de battements cardiaques instables parmi les fenêtres de battements instables.

11. Système (200) selon l'une quelconque des revendications 9 et 10, dans lequel le circuit de commande de présentation (220) est configuré pour déterminer le sous-ensemble cible qui correspond à une fenêtre instable détectée temporellement en premier parmi les fenêtres de battements instables.

12. Système (200) selon l'une quelconque des revendications 9 à 11, dans lequel le circuit de commande de présentation (220) est configuré pour déterminer le sous-ensemble cible qui correspond à une fenêtre instable qui aboutit à une séquence de fenêtres instables consécutives qui excèdent un nombre de seuil de fenêtres instables.

13. Système (200) selon la revendication 12, dans lequel le circuit de commande de présentation (220) est configuré pour détecter, parmi deux séquences ou plus de fenêtres instables consécutives, une séquence de fenêtres instables consécutives la plus longue, et pour déterminer le sous-ensemble cible qui correspond à une fenêtre instable qui aboutit à la séquence la plus longue de fenêtres instables consécutives.

14. Système (200) selon l'une quelconque des revendications 9 à 13, dans lequel le circuit de commande de présentation (220) est en outre configuré pour :
lisser une tendance de comptages de battements cardiaques instables sur les fenêtres de battements ; et
détecter une ou plusieurs fenêtres instables dont chacune comporte le comptage de battements cardiaques instables correspondant, déterminé à partir de la tendance lissée, qui excède le seuil de comptage de battements instables.

15. Système (200) selon la revendication 14, dans lequel le circuit de commande de présentation (220) est configuré pour lisser la tendance du comptage de battements cardiaques instables en utilisant une moyenne mobile de la tendance de comptages de battements cardiaques instables sur les fenêtres de battements.
